# EUROPEAN PATENT SPECIFICATION

(11) **EP 4 277 539 B1**
(45) Date of publication and mention of the grant of the patent: **24.12.2025**
(21) Application number: 22739230.5
(22) Date of filing: 11.01.2022
(51) Int. Cl.: A61B 10/02, A61B 10/00, A61M 31/00

(54) **A DEVICE FOR COLLECTION AND SCAVENGING OF BIOLOGICAL SAMPLES, AND DRUG DELIVERY FOR HOLLOW ORGANS**
VORRICHTUNG ZUM SAMMELN UND SPÜLEN BIOLOGISCHER PROBEN UND ARZNEIMITTELABGABE FÜR HOHLORGANE
DISPOSITIF DE COLLECTE ET DE PIÉGEAGE D'ÉCHANTILLONS BIOLOGIQUES ET ADMINISTRATION DE MÉDICAMENT POUR DES ORGANES CREUX

(30) Priority: 15.01.2021 IN 202141001916
(43) Date of publication of application: 22.11.2023
(73) Proprietor: Thekkedath, Ramachandran, Kerala Pattambi 679303 (IN)
(72) Inventor: Thekkedath, Ramachandran, Kerala Pattambi 679303 (IN)
(74) Representative: Lapienis, Juozas
(86) International application number: PCT/IB2022/050174
(87) International publication number: WO 2022/153171

(56) References cited:
- CN-A- 105 797 251
- CN-U- 213 759 751
- JP-A- H0 972 900
- US-A1- 2009 234 325
- US-A1- 2017 157 369
- US-A1- 2018 193 616
- US-B2- 8 157 767
- US-B2- 9 180 281

## Description

### Priority Claim:

This application claims priority from the provisional application numbered 202141001916 filed with Indian Patent Office, Chennai on 15th January 2021 entitled *"A device for collection and scavenging of biological samples, and drug delivery for hollow organs",* which is provided for reference.

### DESCRIPTION OF THE INVENTION

### Technical field of the invention

The present invention generally relates to a device for the collection and scavenging biological samples, and drug delivery for hollow organs. More particularly, the invention relates to the device for scavenging and collecting the samples and foreign materials such as pathogens including viruses from both lower and upper respiratory tracts as well as hollow organs such as the trachea, esophagus, etc.

### Background of the invention

Several respiratory infections such as seasonal flu affect a significant number of people all over the world. The infective organisms gain entry to the respiratory tract by inhalation of droplets and invade the mucosa destroying the epithelia along with redness, edema, hemorrhage and sometimes an exudate. Moreover, such infections are particularly dangerous for children, older adults and affect individuals with immune system disorders, as the infections vary in severity and require hospitalization in certain individuals. Consequently, necessitating vaccination to prevent severe flu which is further mandated in healthcare professionals and researchers as a precautionary measure. Yet, the efficacy of these vaccines varies with individuals and maybe ineffective with the emergence of new strains of the flu virus. Hence, further necessitating the development of new vaccines to treat emerging new strains of viruses.

Moreover, the recent pandemic situation has created an unprecedented crisis around the world affecting the health and wellbeing of people apart from an enormous strain on the healthcare and economic systems globally. Many of the current diagnostic approaches rely on the collection of a sample using a swab and multiplying the viral content for detection using Polymerase Chain Reaction (PCR) and other methods, which are laborious and time-consuming. Both PCR and rapid detection methods give false-negative results due to errors in sample collection, which further poses an added risk of not addressing the condition during false-negative results. This has raised a need for ensuring proper sample collection practices and a device capable of collecting the sample reliably.

While various research groups around the world are trying to develop vaccines, antiviral medicines and antibodies, there is no definite treatment yet, against viral diseases such as the recent Corona Virus Disease (COVID-19). Moreover, the evolving nature of the novel virus and the emerging indication about the disease has further complicated to validate the safety and efficacy of systemic treatment. Presently, the uncontrolled nature of the pandemic with potential community spread is expected to significantly increase the mortality and will further limit availability and access of healthcare facilities to the needy. The presence of asymptomatic and mildly symptomatic carriers also complicates the matter largely as they mask the presence of viruses or pathogens in them, which may result in the risk of community spread to other members closely associated with such asymptomatic or symptomatic carriers. Although it is essential and a strict rule to maintain good hygiene and social distancing are being practiced for avoiding the spread of the infection, it may be highly impossible in countries with high population density to strictly follow and adhere to the preventive measures. Hence necessitating, a device for efficient viral sample collection and scavenging approach that is capable of reducing the viral load and in turn to reduce the progress of infection. This further aids in reducing the mortality rate thus proving to be a very important and valuable means to overcome the infections associated with the respiratory tract in the present scenario.

The general practice for sample collection involves using cotton or a plastic swab and introducing the same to the infected region such as the upper or lower respiratory tract to collect the pathogen or virus sample. The tip of the swab is used to swipe the surfaces of the nasal cavity or throat and the sample with traces of mucus secretions along with cells if any is transferred to a vial comprising the collection solution. But some of the drawbacks while implementing such an approach are the possibility of absence of the virus, when the virus population is not large enough during the time of sample collection or when the infection is at the initial stages or when the physical contact of the swab to the surface is inadequate. Once these drawbacks are overcome by ensuring proper sample collection technique, which further results in an increased quantity of the collected pathogen, the amplification stage, which is the main time-consuming stage, can be dispensed and expedite the process to give faster and more accurate results. Thus, reduces the chances of false-negative results arising out of the inadequate sample collection.

The Patent Application CN111166391 entitled *"Throat swab collecting device capable of reducing infection risk of novel coronavirus and collecting method thereof*' discloses a medical throat swab specimen collecting device. The device comprises a protective mask, a three-stage sleeve, an external expansion type collecting piece and an air extraction negative pressure device. The device protects medical staff during throat swabs collection from the patients, thus reducing the risk of infection of the medical staff for infectious diseases of a respiratory system. The protective mask is connected with the air exhaust negative pressure device and used for controlling the diffusion of exhaled droplets of a patient. Moreover, the three-stage sleeve is collapsible and can penetrate the throat part of the patient, the contact area is increased after the outward expansion type collecting piece is deformed to collect a sample of the throat part, and then the sample is returned to a cage and stored in the tube to prevent the throat swab sample from being polluted.

The Patent Application CN201555742U entitled *"Sputum sample harvester"* discloses a sputum sample harvester. The harvester comprises a sputum aspirator tube, conduit, negative pressure suction ball, collecting bottle, front end of the conduit is connecting the sputum aspirator tube whose outer wall is provided with scale mark, while the rear end is inhaled ball with negative pressure and linked to each other. The conduit leading portion in wall connects the dividing plate comprising a center pit which further connects the funnel-form joint on dividing plate the front-end tube wall joint and the lower end screw thread collecting bottle.

The Patent Application US20060249161A1 entitled *"Methods and apparatus for nasal aspiration"* disclose an apparatus and methods for withdrawing nasopharyngeal fluid samples from a patient. The invention discloses a catheter and a manual suction device. The catheter is self-navigating and comprises aspiration holes through which aspiration fluid can be emitted and nasopharyngeal fluid sample is withdrawn. The catheter also comprises an insertion depth scale to ensure patient comfort and optimum sample quality. The methods for obtaining nasopharyngeal fluid samples include the steps of inserting the catheter into a patient's nasopharynx and aspirating the nasal pharynx to withdraw a specimen.

The Patent Application AU701703B2 entitled *"Nasopharyngeal wash collection device"* discloses a self-contained manual device for delivering and collecting fluid. The device comprises a container for holding the fluid, a nozzle removably secured to a neck, a pressurizing and depressurizing means, fluid communication means along with a cap to close the neck when the nozzle is removed. The container consists of an opening for the transfer of the fluid to and from the container. The opening is surrounded by the neck, the container also includes a means for pooling the fluid, wherein the pooling means is distally spaced from the opening. While the nozzle is configured to facilitate discharge and collection of the fluid. The fluid communication means extends between the nozzle and the pooling means for providing a flow path for the fluid whereby pressurization of the interior volume results in the discharge of the fluid from the container and depressurization of the interior volume results in aspiration of the fluid into the container.

The Patent Application US8157767B2 entitled *"Devices for cooling the nasal cavity"* discloses a cerebral cooling device that uses a pressurized source to deliver a fluid that evaporates in the nasal cavity to provide cooling and has a balloon on the distal end that inflates from some of the pressure from the pressurized source. The device includes a nasal catheter having delivery ports located in the distal region and a balloon on the distal end. The proximal end of the catheter is in fluid communication with a pressurized source of a low boiling point fluid. A manifold located between the pressurized source and the catheter distributes the fluid and pressure from the pressurized source to a first lumen of the catheter to inflate the balloon and to a second lumen of the catheter through the delivery ports to cool the nasal cavity. A check valve in the manifold ensures that the fluid and pressure are first delivered to the balloon.

The Patent Application US2009234325A1 entitled *"Methods and devices for non-invasive cerebral and systemic cooling"* discloses a method for pharyngeal and cerebral cooling by delivering an ice slurry, slush or super-cooled gel to a patient's nasal cavity, oral cavity and/or throat. In one method, a cooling assembly is inserted into a nasal cavity through a patient's nostril. The cooling assembly includes a flexible balloon defining a chamber and a first elongate tubular member having a lumen in fluid communication with the chamber. A ice slurry having a temperature between about -5° C. and about 5° C. is delivered through the lumen of the first elongate tubular member into the chamber, wherein the flexible balloon expands to place it in contact with the nasal cavity. In another method, an expandable member is inserted through the patient's oral cavity and positioned such that when expanded it forms a low-pressure seal substantially isolating the nasal and oral cavities from the patient's trachea. An ice slurry is delivered directly to the patient's nasal cavity.

Hence, in order to overcome the disadvantages that exist in the state of the art, there is a need for a device for an error-free specimen collection as well as the removal of the virus and other foreign materials from the lower and upper respiratory tract and other hollow organs.

### Summary of the invention

The present invention overcomes the drawbacks of existing biological sample collection devices. The present invention aids in improved sample collection from upper and lower respiratory passages for fast and reliable results. The device eliminates errors or false-negative results.

The present invention discloses a device (100) for the collection of biological samples, scavenging and drug delivery for upper respiratory system. The device is also useful in drug delivery for hollow organs.

The device of the present invention comprises a distal balloon assembly **(101),** a distal balloon **(102),** a proximal balloon assembly **(103),** a proximal balloon **(104)** with a balloon inflation tube **(108),** a double lumen catheter **(105),** a distal balloon **(102),** an agitator **(106)** and a flexible rod **(107).**

The sample collection and scavenging procedure comprise the steps of mounting the distal balloon assembly **(101)** and inflating the distal balloon **(102)** once the proximal balloon assembly **(103)** is in place with the outer tube **(105a)** of the double lumen catheter **(105),** used for infusing and aspirating the liquid within the upper respiratory tract, followed by inflating the proximal balloon **(104),** infusing the collection liquid into the confined cavity, agitating the liquid adequately using the agitator **(106),** aspirating the liquid, transferring it to the collection vial for testing, deflating the proximal balloon **(104)** and distal balloon **(102)** and withdrawing the double lumen catheter **(105)** and distal balloon assembly **(101)** and proximal balloon assembly **(103)** altogether.

The device of the present invention is useful for improved virus collection from upper and lower respiratory passages for fast, reliable, and errorless results. The present invention provides a device or a catheter with balloons to isolate pathogens or virus from the nasal cavity, nasopharynx, or relevant segment of hollow organs. The device and method are thus useful for sample collection and scavenging the viral or pathogens from the nasal cavity as well as for drug delivery inside other hollow organs.

### Brief description of the drawings

The foregoing and other features of embodiments will become more apparent from the following detailed description of embodiments when read in conjunction with the accompanying drawings. In the drawings, like reference numerals refer to like elements.
**Figure** 1 illustrates a device for the collection of biological samples, scavenging and drug delivery for upper respiratory system according to an embodiment of the invention.
**Figure-1A** illustrates a modification of the device for the collection of biological samples, scavenging and drug delivery for upper respiratory system according to another embodiment of the invention.
**Figure 2** illustrates a device for the collection of biological samples, scavenging and drug delivery for lower respiratory system and other hollow organs according to another embodiment of the invention.

### Detailed description of the invention

Reference will now be made in detail to the description of the present subject matter, one or more examples of which are shown in figures.

In order to more clearly and concisely describe and point out the subject matter of the claimed invention, the following definitions are provided for specific terms, which are used in the following written description.

The term *"Swab"* refers to a small piece of soft, absorbent material, such as gauze usually attached to a stick or a wire to aid access, to apply medicine, or take samples of body fluids, as the context requires.

The present invention discloses a single or combination of devices that are useful in collecting and scavenging biological samples and foreign materials from both lower and upper respiratory tracts. The device is also useful in drug delivery for hollow organs.

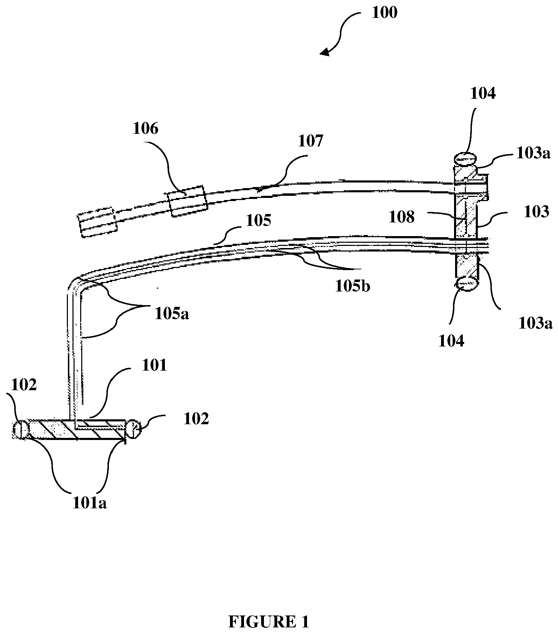

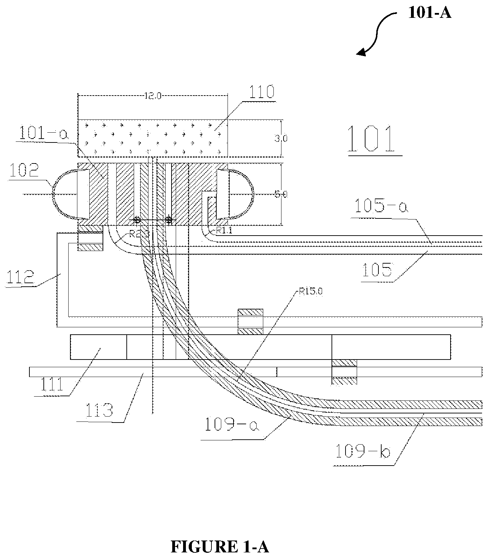

**Figure 1** illustrates a device **(100)** for the collection of biological samples, scavenging and drug delivery for upper respiratory system according to an embodiment of the invention. The device **(100)** comprises a distal balloon assembly **(101),** which comprises a distal carrier disc **(101a),** a distal balloon **(102),** a proximal balloon assembly **(103)** or a nose plug assembly which comprises a proximal carrier disc **(103a),** a proximal balloon **(104)** with a balloon inflation tube **(108),** a double lumen catheter **(105)** with an outer tube **(105a)** and inner tube **(105b)** for inflation of the distal balloon ( **102),** an agitator **(106)** and a flexible rod **(107).**

The distal balloon assembly **(101)** is mounted at the lower end of the nasopharynx region near the soft palate. The proximal balloon assembly **(103)** is mounted inside the nostril. Both the assemblies are mounted watertight by use of the balloons **(102 and 104).** The distal balloon **(102)** is inflated by water or air injected through the inner tube **(105b)** of the double lumen catheter **(105).** The proximal balloon **(104)** is inflated through the balloon inflation tubes **(108)** attached to the proximal balloon assembly **(103).** The agitator **(106)** is mounted on the flexible rod **(107)** passing through the no se plug. The assemblies are mounted in both nostrils, with the double lumen catheter **(105)** connected to distal balloon assembly **(101)** in one nostril.

The sample collection and scavenging procedure comprise the steps of mounting the distal balloon assembly **(101)** and inflating the distal balloon **(102)** once the proximal balloon assembly **(103)** is in place with the outer tube **(105a)** of the double lumen catheter **(105),** used for infusing and aspirating the liquid within the upper respiratory tract, followed by inflating the proximal balloon **(104),** infusing the collection liquid into the confined cavity, agitating the liquid adequately using the agitator **(106),** aspirating the liquid, transferring it to the collection vial for testing, deflating the proximal balloon **(104)** and distal balloon **(102)** and withdrawing the double lumen catheter **(105)** and distal balloon assembly **(101)** and proximal balloon assembly **(103)** altogether.

The agitator **(106)** is flexible and extendable up to nasopharynx and loaded into both the nostril and it comprises an attachment, which mainly aids in physical agitation or scrubbing by to-and-fro movement or rotation or ultrasonication or vibration, pulsation etc. The tool further aids in local heating by conduction or convection or radiation and is capable of applying local medication, provided with a provision for injection and sucking of air instead of water through the agitator **(106)** if situation warrants.

According to another embodiment of the invention, **Figure -1A** illustrates a modification of the device for the collection of biological samples, scavenging and drug delivery for upper respiratory system. The device **(101-A)** comprises the distal balloon assembly **(101),** which is inserted through the mouth using a mounting platform **(111)** for proper positioning of the distal plug **(101a)** which in turn carries the inflated distal balloon **(102).** The distal balloon assembly **(101)** comprises a main collection fluid injection and extraction tube **(105),** a balloon inflation tube **(105a),** a flexible shaft assembly **(109a and 109b) on** which is mounted an agitator or exciter unit **(110),** the mounting platform **(111)** comprising a lever system **(112)** for raising the distal balloon assembly **(101)** once inserted. The distal balloon assembly **(101)** comprises a tray **(113)** made up of any soft material, which is advanced below nasopharynx region after the distal balloon assembly **(101)** is locked in position.

The proximal plug **(103)** is mounted on the nose according to an embodiment of the invention. The distal plug assembly **(101)** comprising the plug **(101a),** the balloon **(102),** the agitator shaft and tubes **(109a, 109b),** the excitor unit **(110),** the balloon inflation tube **(105a)** and the aspirate tube **(105).** The mounting and raising platform **(111)** comprising base plate and lever **(112).** The base plate and the lever **(112)** are inserted through the mouth, placed in a position for raising towards the lower end of nasopharynx using the levers **(112),** and on reaching the suitable elevation, the balloons **(102)** are inflated to achieve a leak tight joint with nasopharynx wall. The mounting platform **(111)** is suitably placed and fixed on and between the upper and lower set of teeth for freeing the hand of the operator. The tray **(113)** is then advanced and placed in the suitable position to collect or soak any leakage of the aspirate fluid. The non-return valves are fitted on the tubes **(105a and 105)** for the aspirate extraction and on the tubes **(105a and 105)** for the inflation of the balloon. Thus, with this assembly, main aspirate extraction and agitator operation are achieved either through the nasal or the oral cavity, preferably through the oral cavity. After positioning the distal balloon **(102),** all other components are mounted and function like the embodiment as described in device **(100).**

The sample collection and scavenging procedure comprises the steps of mounting the distal balloon assembly **(101)** and inflating the distal balloon **(102)** once the proximal balloon assembly **(103)** is held in the initial position. The proximal balloon **(104)** is inflated along with inserting the distal balloon **(101)** and placing the assembly into pharynx through the mouth. The balloon assembly **(101)** is placed at the bottom of the nasopharynx along with inflating the distal balloon **(102),** agitating the liquid adequately using the agitator **(106).** The liquid is aspirated and transferred into the collection vial for testing. The proximal balloon **(104)** and the distal balloon **(102)** are deflated. The distal balloon **(102),** mounting platform **(111)** assembly and the proximal balloon assembly **(103)** are withdrawn.

The exciter unit **(110)** is flexible and extendable up to the top position of nasopharynx and loaded into both the nostrils. The exciter unit **(110)** comprises an attachment, which mainly aids in physical agitation or scrubbing by to-and-fro movement or rotation or ultrasonication or vibration, pulsation etc. The tool further aids in local heating by conduction or convection or radiation and is capable of applying local medication, provided with a provision for injection and sucking of air instead of water through the exciter unit **(110)** if situation warrants. The agitator **(106)** which is mounted on the proximal balloon is available for agitation inside both the nostrils. The injection and extraction of aspirate is done either from mouth or nose depending on the case.

Having generally described this invention, a further understanding can be obtained by reference to certain specific examples, which are provided herein for purposes of illustration only and are not intended to be limiting unless otherwise specified.

### Example 1: Collection and scavenging device for pathogens and foreign materials in the upper respiratory tract

The sample collection and scavenging procedure comprises the steps of removing the device **(100)** from the pouch, mounting the proximal balloon assembly **(103)** in the nostrils and inflating the proximal balloons **(104)** once the distal balloon assembly **(101)** and the double lumen catheter **(105)** are placed in maximum withdrawn condition within the upper respiratory tract, advancing the distal balloon assembly **(101)** and the double lumen catheter **(105)** till the distal balloon **(102)** reaches the nasopharynx bottom and locking the position, inflate the distal balloon **(102)** till it snugly fits in the pharynx opening, tilting the subject's head backwards slightly to give nasal passage a backward downward tilt, introducing the aspirate through the outer tube **(105a)** of the double lumen catheter **(105)** through one of the nostrils to fill the nasal passages, subsequently check the leak tightness of the distal balloon **(102)** at the nasopharynx, followed by agitating the aspirate inside the nasal passages using the agitator **(106),** aspirating the sample solution after reasonable time by tilting the head forward, transferring the sample solution into the collection vial, deflating the distal balloon **(102)** and withdrawing the distal balloon assembly **(101)** and the double lumen catheter **(105),** deflating the nostril sealing proximal balloons **(104)** and finally withdrawing the proximal balloon assembly **(103).** The nasal cavity is cleaned by blowing the nose if required once the sample is collected and the device is removed.

### Example 2: Collection and scavenging device for pathogens and foreign materials in the lower respiratory tract and other hollow organs.

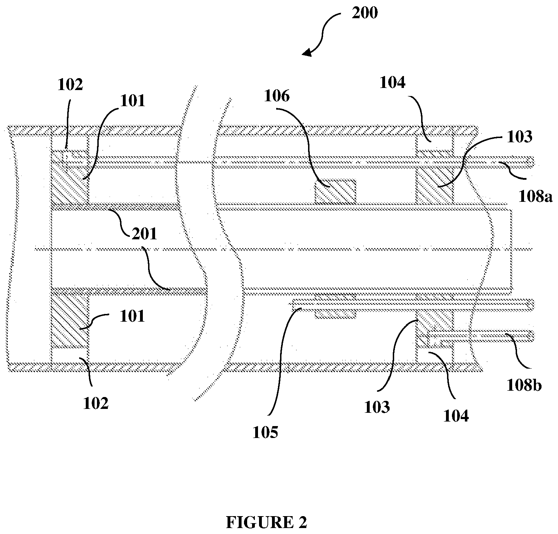

According to yet another embodiment of the invention, the device is used in the trachea and bronchi where the functionality of the organs is maintained by a central open passageway tube. The device is used in hollow organs such as the intestine, esophagus, etc. **Figure 2** illustrates a device **(200)** for the collection of biological samples, scavenging and drug delivery for lower respiratory tract and other hollow organs. The device **(200)** comprises the distal balloon assembly **(101),** distal balloons **(102),** a balloon inflation tubes **(108a** and **108b),** a central open passageway tube **(201),** a proximal balloon catheter assembly **(103),** the proximal balloons **(104),** an agitator **(106)** with or without a motor, providing the functionality as in device **(100),** and catheter **(105)** to aspirate or infuse.

The sample collection and scavenging procedure comprise the steps of removing the device **(200)** from the pouch, setting the position of the proximal balloon **(104)** as per the requirement of the patient, introducing the collection device into the hollow organ such as the trachea, advancing the device till the distal balloon **(102)** reaches an appropriate position in the trachea, inflating the distal balloon **(102)** by means of the balloon inflation tube **(108a),** till the inflated balloon snugly fits in the trachea, followed by inflating the proximal balloon **(104)** by means of the balloon inflation tube **(108b),** subsequently introducing the aspirate by means of the catheter **(105)** and agitating using the agitator **(106),** aspirating the sample solution after a reasonable time, transferring the sample solution into the collection vial for testing, finally deflating the balloons **(102** and **104)** before withdrawing the device **(200)** assembly.

The advantages of using the device and technique are improved pathogen or virus collection from upper and lower respiratory passages for fast, precise and reliable results. It eliminates errors or false-negative results particularly in respiratory infections including COVID-19 testing. Moreover, the localized approach of scavenging most of the viruses from respiratory passages, that are the main areas where the viruses are primarily located in early stage prevents the development of the disease, as well as its manifestation into severe disease. Thus, the device of the present invention further aids in reducing the spread of infection to potential contacts. Additionally, reducing the community spread from asymptomatic carriers and reducing the severity of the condition in a significant number of patients in the early stages of the disease, which further reduces the anticipated load on the healthcare system and associated resources.

Thus, the present invention provides a device or a catheter with balloons to isolate pathogens or virus from the nasal cavity, nasopharynx, or relevant segment of hollow organs. Further, the use of sample collection liquid that comes in contact with all relevant surfaces to collect the sample proves to be an error-free approach. Moreover, the sample collection efficiency is improved by the agitation using various techniques such as ultrasonication, vibration, to-and-fro movement of the liquid apart and also other physical and chemical techniques which result in increasing the secretion of mucus. Thereby, ensuring the release and collection of the viruses or other pathogens within the cells and or in the extracellular space. The technique is useful in reducing or removal of the virus or pathogen. The device and method are thus useful for sample collection and scavenging the viral or pathogens from the nasal cavity as well as for drug delivery inside other hollow organs.

### Reference numbers:

| **Components** | **Reference Numbers** |
|---|---|
| Biological sample collecting and scavenging device | **100** |
| Distal Balloon Assembly | **101** |
| Distal Carrier Disc or Distal Plug | **101a** |
| Distal Balloon | **102** |
| Proximal Balloon Assembly or Nose Plug Assembly | **103** |
| Proximal Carrier Disc | **103a** |
| Proximal Balloon | **104** |
| Double Lumen Catheter or Aspirate Tube | **105** |
| Inner Tube | **105a** |
| Outer Tube | **105b** |
| Agitator | **106** |
| Flexible Rod | **107** |
| Balloon Inflation Tube | **108** |
| Balloon Inflation Tube | **108a** |
| Balloon Inflation Tube | **108b** |
| Flexible Shaft Assembly | **109a and 109b** |
| Excitor Unit | **110** |
| Mounting Platform | **111** |
| Lever System | **112** |
| Tray | **113** |

## Claims

1. A device for collection of one or more biological samples, scavenging and for drug delivery in the upper respiratory tract, the device **(100)** comprising:
a) a distal balloon assembly **(101)** comprising a distal carrier disc **(101a)** and a distal balloon **(102),** the distal balloon assembly **(101)** configured to be mounted watertight by the distal balloon **(102)** at the lower end of nasopharynx region above the soft palate;
b) a proximal balloon assembly or nose plug assembly **(103)** comprising proximal carrier disc **(103a),** a proximal balloon **(104)** and a balloon inflation tube **(108),** the proximal balloon assembly or the nose plug assembly **(103)** configured to be mounted watertight by the proximal balloon **(104)** inside a nostril;
c) a double lumen catheter **(105)** connected to the distal balloon **(102),** configured to be placed in one of the nostrils and configured to inflate the distal balloon **(102)** by injecting air or water;
the device **(100) characterized in that** an agitator **(106)** is mounted on a flexible rod **(107)** through the nose plug assembly (103), and is configured to be used for agitation of a liquid into a confined cavity.

2. The device as claimed in claim 1, wherein said distal balloon assembly **(101)** is configured to be inserted through the mouth accessing the nasopharynx region of the user.

3. The device as claimed in claim 1, wherein the double lumen catheter **(105)** further comprises an outer tube **(105a)** and an inner tube **(105b).**

4. The device as claimed in claim 1, wherein said distal balloon **(102)** is configured to be inflated by water or air and the proximal balloon **(104)** is configured to be inflated through one or more balloon inflation tubes **(108a and 108b).**

5. The device as claimed in claim 1, wherein said distal balloon assembly **(101)** is supported laterally at the soft palette with one or more attachments.

6. The device as claimed in claim 1, wherein device **(101-A)** for insertion through the mouth further comprises:
a. a flexible shaft assembly **(109)** comprising an agitator shaft **(109a)** and an agitator tube **(109b);**
b. an excitor unit **(110)** configured to be mounted on the flexible shaft assembly **(109);**
c. a mounting platform **(111)** comprising a raising platform and a lever system **(112)** configured to raise the distal balloon assembly **(101)** after insertion; and
d. a tray **(113)** placed below the nasopharynx region configured to collect the aspirate fluid.

7. The device as claimed in claim 6, wherein said device **(101-A)** comprising the distal balloon **(102)** is configured to be inflated to achieve leak tight joint with the nasopharynx wall.

8. The device as claimed in claim 6, wherein said device **(101-A)** comprising the excitor unit **(110)** is extendable up to top portion of the nasopharynx region and aids in physical agitation.

9. The device as claimed in claim 6, wherein said device **(101-A)** comprising the mounting platform **(111)** configured to be mounted between upper and lower set of teeth inside the mouth.

10. The device as claimed in claim 6, wherein said device **(101-A)** comprising a base plate and the lever **(112)** configured to be inserted through the mouth and raised towards lower end of the nasopharynx using the levers **(112).**

11. The device as claimed in claim 1, wherein device **(200)** further comprises a central open passageway tube **(201)** which maintains the functionality of the organs and collects one or more biological samples, scavenges and aids in drug delivery to hollow organs in the lower respiratory tract.

## Patentansprüche

1. Vorrichtung zum Sammeln von einer oder mehreren biologischen Proben, zur Reinigung und zur Arzneimittelabgabe im oberen Atemtrakt, wobei die Vorrichtung **(100)** umfasst:
a) eine distale Ballonbaugruppe **(101),** die eine distale Trägerscheibe **(101a)** und einen distalen Ballon **(102)** umfasst, wobei die distale Ballonbaugruppe **(101)** so konfiguriert ist, dass sie durch den distalen Ballon **(102)** am unteren Ende des Nasopharynxbereichs oberhalb des weichen Gaumens wasserdicht montiert wird;
b) eine proximale Ballonbaugruppe oder Nasenstopfenbaugruppe **(103),** die eine proximale Trägerscheibe **(103a),** einen proximalen Ballon **(104)** und ein Ballonaufblasrohr **(108)** umfasst, wobei die proximale Ballonbaugruppe oder die Nasenstopfenbaugruppe **(103)** so konfiguriert ist, dass sie durch den proximalen Ballon **(104)** wasserdicht in einem Nasenloch montiert wird;
c) einen mit dem distalen Ballon **(102)** verbundenen Doppellumenkatheter **(105),** der so konfiguriert ist, dass er in eines der Nasenlöcher platziert wird, und so konfiguriert ist, dass er den distalen Ballon **(102)** durch Einspritzen von Luft oder Wasser aufbläst;
die Vorrichtung **(100) dadurch gekennzeichnet ist, dass** ein Rührwerk **(106)** durch die Nasenstopfenbaugruppe (103) an einer flexiblen Stange **(107)** montiert und so konfiguriert ist, dass es zum Rühren einer Flüssigkeit in einem begrenzten Hohlraum verwendet wird.

2. Vorrichtung nach Anspruch 1, wobei die genannte distale Ballonbaugruppe **(101)** so konfiguriert ist, dass sie durch den Mund eingeführt wird und den Nasopharynxbereich des Benutzers erreicht.

3. Vorrichtung nach Anspruch 1, wobei der Doppellumenkatheter **(105)** zusätzlich ein Außenrohr **(105a)** und ein Innenrohr **(105b)** umfasst.

4. Vorrichtung nach Anspruch 1, wobei der genannte distale Ballon **(102)** so konfiguriert ist, dass er mit Wasser oder Luft aufgeblasen wird, und der proximale Ballon **(104)** so konfiguriert ist, dass er durch ein oder mehrere Ballonaufblasrohre **(108a und 108b)** aufgeblasen wird.

5. Vorrichtung nach Anspruch 1, wobei die genannte distale Ballonbaugruppe **(101)** seitlich am weichen Gaumen mit einer oder mehreren Befestigungen abgestützt ist.

6. Vorrichtung nach Anspruch 1, wobei eine Vorrichtung **(101-A)** zur Einführung durch den Mund zusätzlich umfasst:
a. eine flexible Wellenbaugruppe **(109),** die eine Rührwerkswelle **(109a)** und ein Rührwerksrohr **(109b)** umfasst;
b. eine Erregereinheit **(110),** die so konfiguriert ist, dass sie auf einer flexiblen Wellenbaugruppe **(109)** montiert wird;
c. eine Montageplattform **(111),** die eine Hubplattform und ein Hebelsystem **(112)** umfasst, das so konfiguriert ist, dass es die distale Ballonbaugruppe **(101)** nach der Einführung anhebt; und
d. eine Schale **(113),** die unterhalb des Nasopharynxbereichs angeordnet und so konfiguriert ist, dass sie die aspirierte Flüssigkeit sammelt.

7. Vorrichtung nach Anspruch 6, wobei die genannte Vorrichtung **(101-A),** die den distalen Ballon **(102)** umfasst, so konfiguriert ist, dass sie aufgeblasen wird, um leckdichte Verbindung mit der Wand des Nasopharynx herzustellen.

8. Vorrichtung nach Anspruch 6, wobei die genannte Vorrichtung **(101-A),** die die Erregereinheit **(110)** umfasst, bis zum oberen Teil des Nasopharynxbereichs ausziehbar ist und das physische Rühren unterstützt.

9. Vorrichtung nach Anspruch 6, wobei die genannte Vorrichtung **(101-A)** die Montageplattform **(111)** umfasst, die so konfiguriert ist, dass sie zwischen der oberen und der unteren Zahnreihe innerhalb des Mundes montiert wird.

10. Vorrichtung nach Anspruch 6, wobei die genannte Vorrichtung **(101-A)** eine Grundplatte und den Hebel **(112)** umfasst, die so konfiguriert sind, dass sie durch den Mund eingeführt und mithilfe der Hebel **(112)** zum unteren Ende des Nasopharynx angehoben werden.

11. Vorrichtung nach Anspruch 1, wobei eine Vorrichtung **(200)** zusätzlich ein zentrales offenes Durchgangsrohr **(201)** umfasst, das die Funktion der Organe aufrechterhält und eine oder mehrere biologische Proben sammelt, reinigt und die Arzneimittelabgabe an Hohlorgane im unteren Atemtrakt unterstützt.

## Revendications

1. Un dispositif de prélèvement d'un ou plusieurs échantillons biologiques, d'élimination et d'administration de médicaments dans les voies respiratoires supérieures, le dispositif **(100)** comprenant:
a) un ensemble **(101)** de ballonnet distal comprenant un disque **(101a)** porteur distal et un ballonnet **(102)** distal, l'ensemble **(101)** de ballonnet distal étant configuré pour être monté de manière étanche par le ballonnet **(102)** distal à l'extrémité inférieure de la région du nasopharynx au-dessus du palais mou;
b) un ensemble de ballonnet proximal ou un ensemble **(103)** de bouchon nasal comprenant un disque **(103a)** porteur proximal, un ballonnet **(104)** proximal et un tube **(108)** de gonflage du ballonnet, l'ensemble de ballonnet proximal ou l'ensemble **(103)** de bouchon nasal étant configuré pour être monté de manière étanche par le ballonnet **(104)** proximal à l'intérieur d'une narine;
c) un cathéter **(105)** à double lumière relié au ballonnet **(102)** distal, configuré pour être placé dans l'une des narines et configuré pour gonfler le ballonnet **(102)** distal en injectant de l'air ou de l'eau;
le dispositif **(100) est caractérisé en ce qu'**un agitateur **(106)** est monté sur une tige **(107)** flexible à travers l'ensemble **(103)** du bouchon nasal et est configuré pour être utilisé pour l'agitation d'un liquide dans une cavité confinée.

2. Le dispositif selon la revendication 1, dans lequel ledit ensemble **(101)** de ballonnet distal est configuré pour être inséré par la bouche accédant à la région du nasopharynx de l'utilisateur.

3. Le dispositif selon la revendication 1, dans lequel le cathéter **(105)** à double lumière comprend en outre un tube extérieur **(105a)** et un tube intérieur **(105b).**

4. Le dispositif selon la revendication 1, dans lequel ledit ballonnet **(102)** distal est configuré pour être gonflé par de l'eau ou de l'air et le ballonnet proximal **(104)** est configuré pour être gonflé par un ou plusieurs tubes de gonflage de ballonnet **(108a et 108b).**

5. Le dispositif selon la revendication 1, dans lequel ledit ensemble **(101)** de ballonnet distal est soutenu latéralement au niveau du palais mou par une ou plusieurs fixations.

6. Le dispositif selon la revendication 1, dans lequel le dispositif **(101-A)** destiné à être inséré par la bouche comprend en outre:
a. un ensemble **(109)** d'arbre flexible comprenant un arbre **(109a)** d'agitateur et un tube **(109b)** d'agitateur;
b. une unité **(110)** d'excitation configurée pour être montée sur l'ensemble **(109)** d'arbre flexible;
c. une plateforme **(111)** de montage comprenant une plateforme de levage et un système **(112)** de levier configuré pour soulever l'ensemble **(101)** ballonnet distal après insertion; et
d. un plateau **(113)** placé sous la région du nasopharynx configuré pour recueillir le liquide aspiré.

7. Le dispositif selon la revendication 6, dans lequel ledit dispositif **(101-A)** comprenant le ballonnet **(102)** distal est configuré pour être gonflé afin d'obtenir un joint étanche avec la paroi du nasopharynx.

8. Le dispositif selon la revendication 6, dans lequel ledit dispositif **(101-A)** comprenant l'unité **(110)** d'excitation est extensible jusqu'à la partie supérieure de la région du nasopharynx et contribue à l'agitation physique.

9. Le dispositif selon la revendication 6, dans lequel ledit dispositif **(101-A)** comprend la plateforme **(111)** de montage configurée pour être montée entre les dents supérieures et inférieures à l'intérieur de la bouche.

10. Le dispositif selon la revendication 6, dans lequel ledit dispositif **(101-A)** comprend une plaque de base et le levier **(112)** configuré pour être inséré par la bouche et relevé vers l'extrémité inférieure du nasopharynx à l'aide des leviers **(112).**

11. Le dispositif selon la revendication 1, dans lequel le dispositif **(200)** comprend en outre un tube **(201)** de passage ouvert central qui maintient la fonctionnalité des organes et recueille un ou plusieurs échantillons biologiques, récupère et aide à l'administration de médicaments aux organes creux des voies respiratoires inférieures.
